# EUROPEAN PATENT APPLICATION

(11) **EP 1 275 653 A1**
(43) Date of publication of application: **15.01.2003**
(21) Application number: 01420155.2
(22) Date of filing: 10.07.2001
(51) Int. Cl.: C07D 498/04, A01N 43/90

(54) **Oxazolopyridines and their use as fungicides**

(71) Applicant: Bayer CropScience S.A., 69009 Lyon (FR)
(72) Inventor: Muller, Benoit, 69002 Lyon (FR); Hartmann, Benoit, 69110 Sainte-Foy-Les-Lyon (FR)

(57) **Abstract**

Compounds of general formula (I) 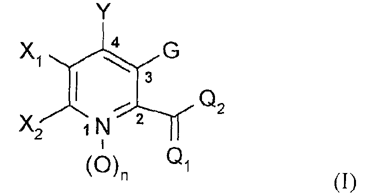 in which:
- Y and G together with carbon atoms 3 and 4, form a 5 or 6 member ring chosen from the following structures A to O : 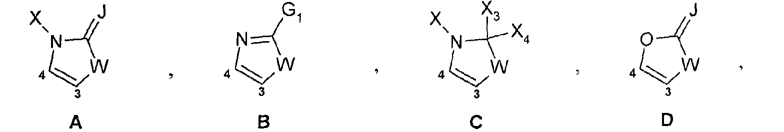 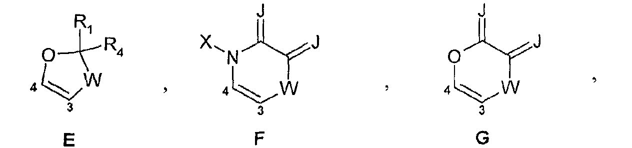 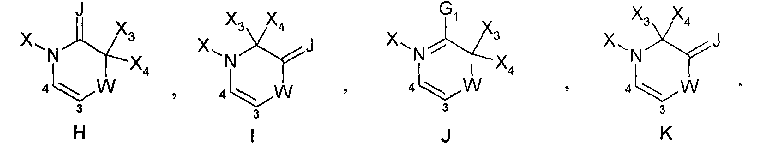 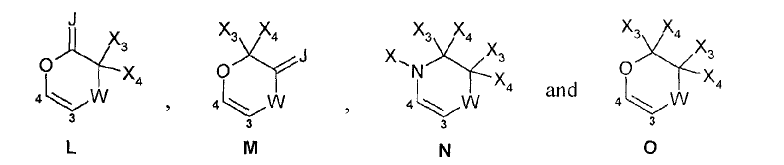
the other substituants being as defined in the description,
process for preparing these compounds,
fungicidal compositions comprising these compounds,
processes for treating plants by applying these compounds or compositions.

## Description

The present invention relates to new derivatives of picolinic acid, their process of preparation, their use as fungicides, particularly in the form of fungicidal compositions, and methods for the control of phytopathogenic fungi of plants using these compounds or their compositions.

Picolinic acid derivatives with fungicidal action are known in the literature. Thus, antimycin and certain derivatives thereof, disclosed in particular in patent application WO-A-99/11127 and by Kuzo Shibata et al. (*The Journal of Antibiotics,* **51** (12), (1998), 1113-1116), are presented as being effective against phytopathogenic fungi of plants, with good efficacy. These compounds, and those disclosed in patent US-A-3 228 950, have no substituants in position 4 of the pyridine nucleus.

Patent application WO-A-00/26191 presents picolinamide derivatives that are optionally substituted in position 4 with a methoxy radical. Patent application WO-A-95/25723 proposes 3-pyridylcarboxylic acid derivatives.

However, these known compounds have the drawback of being toxic products, which forbids any use of these compounds in agriculture for eradicating phytopathogenic diseases of crops. Furthermore, these compounds are obtained from fermentation musts and have relatively complex chemical structures. Thus, the preparations and purifications of these compounds remain demanding and expensive operations, making any industrial synthesis or marketing economically non-viable.

Picolinamide derivatives are also known from patent application JP-11 228 542. These derivatives are presented as having potential antifungal activities and low toxicity, for use in pharmaceutical products. Other picolinic acid derivatives are also known from patent application EP-A-0 690 061, in which such compounds are used as synthetic intermediates for the preparation of pyridothiadiazoles.

Thus, a first object of the present invention comprises a new family of picolinic acid derivatives not possessing previously cited disadvantages.

Another object of the present invention is to propose a new family of compounds having an improved activity, both quantitatively and qualitatively, in comparison with the known fungicides, antimycin or derivatives. By activity, is meant fungicidal activity; by quantitative improvement, is understood a better control of phytopathogenic fungi on plants and, by qualitative improvement, a broader spectrum of activity, that is to say control of a greater variety of phytopathogenic fungi of plants.

Another object of the present invention is to provide a new family of compounds having an improved toxicity and/or phytotoxicity and/or ecotoxicity in comparison with known fungicides, particularly antimycin and its derivatives.

Another object of the present invention is to provide a new family of compounds possessing the characteristics indicated above, notably on crops such as cereals, rice, corn, fruit trees, woodland trees, vines, oil-yielding plants, protein-yielding plants, market garden crops, solanaceous crops, beet, flax, citrus fruits, banana, ornamental plants and tobacco.

Another object of the present invention is to provide a new family of picolinic acid derivatives possessing the characteristics indicated above, notably on crops such as cereals, rice, corn, fruit trees, woodland trees, vine, oil-yielding plants, protein-yielding plants, market garden plants, solanaceous plants, beet, flax, citrus fruits, banana and ornamental plants and tobacco.

Another object of the present invention consists equally in proposing a new family of picolinic acid derivatives useful for the treatment and protection of lumber against fungal disease. In effect, lumber used for example for the fabrication of buildings and furniture (framework, walls, ceilings, flooring etc.), may suffer different injuries, among others, due to phytopathogenic fungi. The compounds according to the present invention allow these attacks to be combated.

Another object of the present invention comprises proposing a new family of picolinic acid derivatives useful in human and animal therapy. In fact, by virtue of their antifungal properties, the picolinic acid derivatives which are the object of the present invention can prove useful for the treatment, in man and animals, of fungal diseases, such as for example mycosis and candidiasis.

Surprisingly it has been found that these objects can be achieved in whole or in part, by picolinic acid derivatives such as those described in the present invention.

### General Definition of Invention

The invention relates to picolinic acid derivatives of general formula (I): 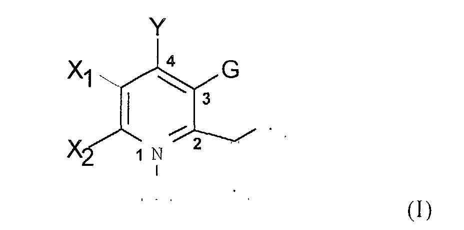 in which:
- Y and G together with carbon atoms 3 and 4, form a 5 or 6 member ring chosen from the following structures A to O: 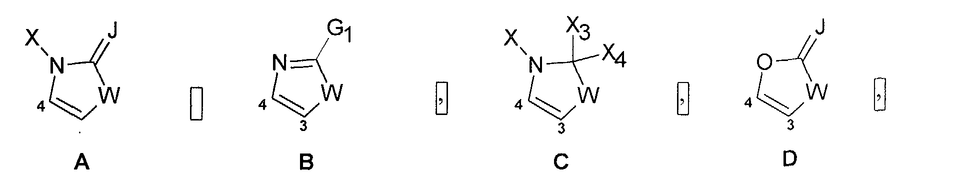 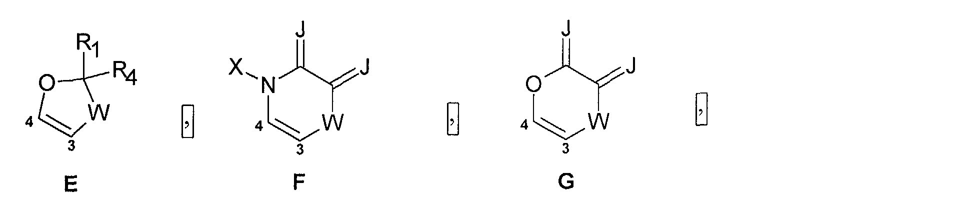 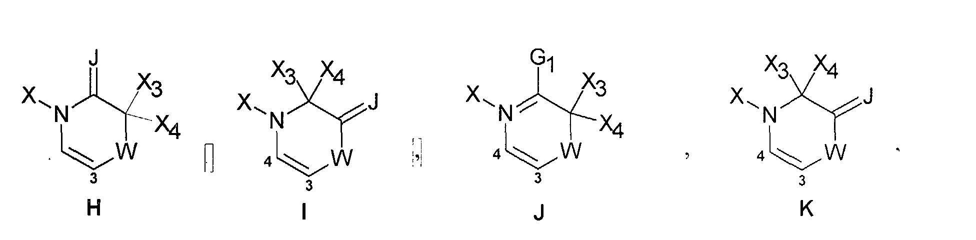 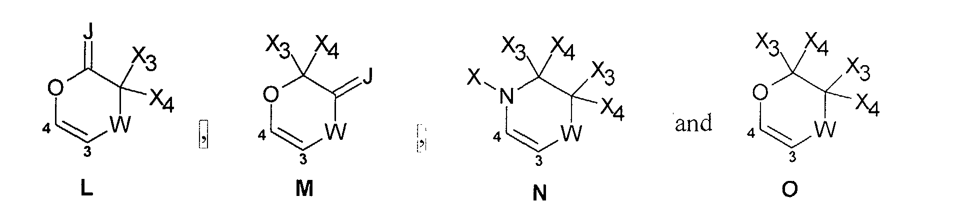
in which:
- J independently represents oxygen or sulphur,
- n represents 0 or 1,
- W is chosen from oxygen and sulphur,
- Q₁ is chosen from oxygen, sulphur, -NR₁ and -N-NR₄R₅,
- Q₂ is chosen from -OR₂, -SR₃ and -NR₄R₅, or
- Q₁ and Q₂ may together form a ring having from 5 to 7 atoms containing 2 to 3 oxygen and/or nitrogen atoms, optionally substituted with one or more radicals, which may be identical or different, chosen from halogens and alkyl and haloalkyl radicals,
- G₁ is chosen from hydrogen, halogen, hydroxy, mercapto, nitro, thiocyanato, cyano or pentafluorosulphonyl, alkyl, haloalkyl, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl, alkoxysulphonyl, cycloalkyl, halocycloalkyl, alkenyl, alkynyl, alkenyloxy, alkynyloxy, alkenylthio, alkynylthio, allyl, aryl, arylalkyl, propargyl, amino, N-alkylamino, N,N-dialkylamino, -NHCOR₁₀, -NHCSR₁₀, N-alkylaminocarbonylamino, N,N-dialkylaminocarbonylamino, aminoalkyl, N-alkylaminoalkyl, N,N-dialkylaminoalkyl, acylaminoalkyl, thioacylamino, alkoxythiocarbonylamino, N-alkylaminothiocarbonylamino, N,N-dialkylaminothiocarbonylamino, N,N-arylalkylaminocarbonylamino, N-alkylsulphinylamino, N-alkylsulphonylamino, N-alkyl(alkylsulphonyl)amino, N-arylsulphinylamino, N-arylsulphonylamino, N-alkoxysulphonylamino, N-alkoxysulphinylamino, N-haloalkoxysulphinylamino, N-haloalkoxysulphonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino, N,N-arylalkylaminothiocarbonylamino, acyl, carboxyl, carbamoyl, N-alkylcarbamoyl, N,N-dialkylcarbamoyl, lower alkoxycarbonyl, N-arylcarbamoyl, N,N-diarylcarbamoyl, aryloxycarbonyl, N,N-arylalkylcarbamoyl, and an imino group of formula: 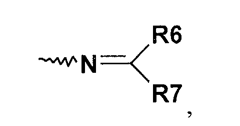
- X is chosen from hydrogen, alkyl, haloalkyl, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl, alkoxysulphonyl, cycloalkyl, halocycloalkyl, alkenyl, alkynyl, alkenyloxy, alkynyloxy, alkenylthio, alkynylthio, allyl, aryl, arylalkyl, propargyl, acyl, carboxyl, carbamoyl, aryloxycarbonyl,
- X₁ and X₂ are identical or different and chosen, independently of each other, from hydrogen, halogen, hydroxy, mercapto, nitro, thiocyanato, azido, cyano, pentafluorosulphonyl, alkyl, haloalkyl, alkoxy, haloalkoxy, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl, and alkoxysulphonyl, or
- X₁ and X₂ may also be joined together, thus forming a saturated, partially unsaturated or totally unsaturated 4- to 8-membered ring optionally comprising one or more heteroatoms chosen from sulphur, oxygen, nitrogen and phosphorus,
- X₃, X₄ are identical or different and chosen, independently of each other, from hydrogen, optionally substituted alkyl comprising from 1 to 12 carbon atoms in a linear or branched chain, hydroxy, amino, haloalkyl, cycloalkyl, halocycloalkyl, alkoxy, aryloxy, arylalkoxy, haloalkoxy, alkylthio, haloalkylthio, alkoxyalkyl, haloalkoxyalkyl, alkylthioalkyl, haloalkylthioalkyl, cyanoalkyl, acyl, nitro, cyano, carboxyl, carbamoyl, 3-oxetanyloxycarbonyl, N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl, N,N-dialkylaminoalkyl radical, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
- X₃ and X₄ may be joined together, thus forming a saturated, partially unsaturated or totally unsaturated 4- to 8-membered ring optionally comprising one or more heteroatoms chosen from sulphur, oxygen, nitrogen and phosphorus,
- R₂ and R₃ are identical or different and chosen, independently of each other, from alkyl comprising from 1 to 12 carbon atoms, haloalkyl, cycloalkyl, halocycloalkyl, alkoxy, haloalkoxy, alkylthio, haloalkylthio, alkoxyalkyl, haloalkoxyalkyl, alkylthioalkyl, haloalkylthioalkyl, cyanoalkyl, acyl, nitro, cyano, carboxyl, carbamoyl, 3-oxetanyloxycarbonyl, and N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl, and N,N-dialkylaminoalkyl radical, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
- R₁, R₄, R₅, R₆ and R₇ are identical or different and chosen, independently of each other, from hydrogen, optionally substituted alkyl comprising from 1 to 12 carbon atoms in a linear or branched chain, haloalkyl, cycloalkyl, halocycloalkyl, alkoxy, aryloxy, arylalkoxy, haloalkoxy, alkylthio, haloalkylthio, alkoxyalkyl. haloalkoxyalkyl, alkylthioalkyl, haloalkylthioalkyl, cyanoalkyl, acyl, nitro, cyano, carboxyl, carbamoyl, 3-oxetanyloxycarbonyl, and N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl, N,N-dialkylaminoalkyl, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
- R₄ and R₅, on the one hand, or R₆ and R₇, on the other hand, may be joined together, thus forming a saturated, partially unsaturated or totally unsaturated 4-to 8-membered ring optionally comprising one or more heteroatoms chosen from sulphur, oxygen, nitrogen and phosphorus,
- T represents a direct bond or a divalent radical chosen from a radical -(CH₂)ₘ-, m taking a value between 1 and 12, limits included, said radical being optionally interrupted or ending with one or two heteroatoms chosen from nitrogen, oxygen and/or sulphur, and an oxyalkylene, alkoxyalkylene, carbonyl (-CO-), oxycarbonyl (-O-CO-), carbonyloxy (-CO-O-), sulphinyl (-SO-), sulphonyl (-SO2-), oxysulphonyl (-O-SO₂-), sulphonyloxy (-SO₂-O-), oxysulphinyl (-O-SO-), sulphinyloxy (-SO-O-), thio (-S-), oxy (-O-), vinyl (-C=C-), ethynyl (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- or -O-NR₉-CO- radical,
- R₈ is chosen from hydrogen and aryl and heterocyclyl,
- R₉ and R₁₀, which may be identical or different, are chosen, independently of each other, from hydrogen, halogen, hydroxy, mercapto, nitro, thiocyanato, azido, cyano, pentafluorosulphonyl, alkyl, haloalkyl, alkoxy, haloalkoxy, alkylthio, haloalkylthio, alkoxyalkyl, haloalkoxyalkyl, alkylthioalkyl, haloalkylthioalkyl, arylalkyl, cyanoalkyl, cyanoalkoxy, cyanoalkylthio, alkylsulphinyl, haloalkylsulphinyl, alkylsulphonyl, haloalkylsulphonyl, and alkoxysulphonyl,
as well as any optional N-oxides, geometrical and/or optical isomers, enantiomers and/or diastereoisomers, tautomeric forms, salts and metal and metalloid complexes of the compounds of formula (I) as here-above defined.

The tautomeric forms of the compounds of formula (I) such as those defined above are also included in the invention. By tautomeric forms there are to be understood all of the isomeric forms described in the work "The Tautomerism of Heterocycles, Advances in Heterocyclic Chemistry, Supplement 1, by J Elguero, C. Martin, A.R. Katritsky and P Linda, published by Academic Press, New York, 1976, pages 1-4.

The following generic terms are used with the following meanings:
- halogen means fluorine, chlorine, bromine or iodine,
- alkyl radicals as well as groups including these alkyl radicals (alkoxy, alkylcarbonyl or acyl, etc.) include, unless otherwise indicated, from 1 to 6 carbons atoms in a linear or branched chain and are optionally substituted,
- halogenated alkyl, alkoxy and halocycloalkyl radicals may comprise one or more identical or different halogen atoms,
- cycloalkyl radicals comprise from 3 to 6 carbon atoms and are optionally substituted,
- alkenyl and alkynyl radicals, as well as groups including such radicals, comprise, unless otherwise indicated, from 2 to 6 carbon atoms in a straight or branched chain and are optionally substituted,
- acyl means alkylcarbonyl or cycloalkylcarbonyl, the alkyl part of which containing from 1 to 6 carbon atoms and the cycloalkyl part of which containing 3 to 6 carbon atoms, unless otherwise indicated and are optionally substituted,
- alkylene means the divalent -(CH₂)ₘ- radical where m represents an integer equal to 1, 2, 3, 4, 5 or 6,
- the term "aryl" in "aryl" and "arylalkyl" signifies phenyl or naphthyl, optionally substituted,
- the term "heterocyclyl" in "heterocyclyl" and "heterocyclylalkyl" signifies a ring of 4 to 10 members, saturated, partially unsaturated or unsaturated, optionally substituted, comprising one or more heteroatoms, identical or different, chosen from nitrogen, oxygen, sulphur, silicon and phosphorus,
- when the amino radical is disubstituted, the two substituants are identical or different or may together with the nitrogen atom which carries them form a saturated, partially unsaturated or unsaturated nitrogen-containing heterocycle, containing 5 or 6 atoms in total,
- when the carbamoyl radical is disubstituted, the two substituants are identical or different or may together with the nitrogen atom which carries them form a saturated, partially unsaturated or unsaturated nitrogen-containing heterocycle of 5 to 6 carbon atoms in total,
- unless otherwise specified, the expression "optionally substituted" qualifying an organic group applies to different radicals constituting the group and indicates that the different radicals are optionally substituted by one or more radicals R₉ and/or aryl and/or arylalkyl, identical or different.

According to one variant of the present invention, the invention relates to picolinic acid derivatives of general formula (I) as defined above and for which:
- X₁ and X₂ each represent a hydrogen atom,
- the other substituants being as defined above,
as well as the optional N-oxides, geometrical and/or optical isomers, enantiomers and/or diastereoisomers, tautomeric forms, salts and metal and metalloid complexes thereof.

According to another aspect, the present invention relates to picolinic acid derivatives of general formula (I) having the following characteristics:
- Q₁ is chosen from oxygen and sulphur,
- the other substituants being as defined above,
as well as optional N-oxides, geometrical and/or optical isomers, enantiomers and/or diastereoisomers, tautomeric forms, salts and metal and metalloid complexes thereof.

According to another aspect, the present invention relates to picolinic acid derivatives of general formula (I) having the following characteristics:
- Y and G together with carbon 3 and 4, form a ring chosen from structures A to E as described herein before,
- the other substituants being as defined above,
as well as the optional N-oxides, geometrical and/or optical isomers, enantiomers and/or diastereoisomers, tautomeric forms, salts and metal and metalloid complexes thereof.

According to a further aspect, the present invention relates to picolinic acid derivatives of general formula (I) as defined above and for which:
- Y and G together with carbon 3 and 4, forms a 5 member ring chosen from : 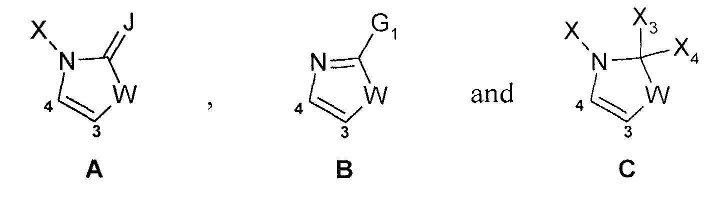
- the other substituants being as defined above,
as well as the optional N-oxides, geometrical and/or optical isomers, enantiomers and/or diastereoisomers, tautomeric forms, salts and metal and metalloid complexes thereof.

According to yet another variant of the present invention, this invention relates to picolinic acid derivatives of general formula (I) as defined above and for which:
- Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl, alkynyl radical and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
- the other substituants being as defined above,
as well as the optional N-oxides, geometrical and/or optical isomers, enantiomers and/or diastereoisomers, tautomeric forms, salts and metal and metalloid complexes thereof.

More particularly, the present invention relates to picolinic acid derivatives of general formula (I) as defined above, which have the following characteristics, taken separately or in combination:
- X₁ and X₂ each represent hydrogen,
- Y and G together with carbon 3 and 4, forms a 5 member ring chosen from : 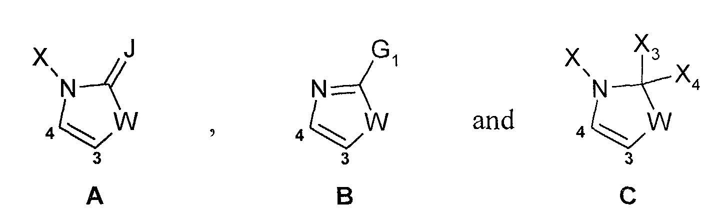
- Q₁ is chosen from oxygen and sulphur,
- Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl and alkynyl radical and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
- the other substituants being as defined above,
as well as the optional N-oxides, geometrical and/or optical isomers, enantiomers and/or diastereoisomers, tautomeric forms, salts and metal and metalloid complexes thereof.

Even more specifically, the present invention relates to picolinic acid derivatives of general formula (I) as defined above, which have the following characteristics:
- X₁ and X₂ each represent hydrogen,
- Y and G together with carbon 3 and 4, forms a 5 member ring chosen from : 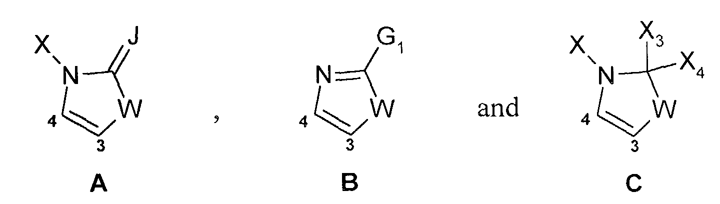
- G1 is chosen from alkyl, hydrogen, and -N,N-dialkylamino,
- Q₁ is chosen from oxygen and sulphur,
- Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl and alkynyl and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
- the other substituants being as defined above,
as well as the optional N-oxides, geometrical and/or optical isomers, enantiomers and/or diastereoisomers, tautomeric forms, salts and metal and metalloid complexes thereof.

In the context of the present invention, the term "aryl" means phenyl or naphthyl, the term "arylalkyl" means phenylalkyl or naphthylalkyl, more particularly benzyl, phenethyl, phenylpropyl, phenylbutyl, naphthylmethyl, naphthylethyl, naphthylpropyl or naphthylbutyl. It is understood that these various radicals may optionally be substituted with one or more radicals R₉ and/or aryl and/or arylalkyl radicals, which may be identical or different.

The terms "heterocyclyl" and "heterocyclylalkyl" are defined similarly, it being understood that "heterocycle" means a saturated, partially unsaturated or unsaturated monocycle or bicycle containing from 4 to 10 atoms, comprising at least one heteroatom chosen from nitrogen, oxygen, sulphur, silicon and phosphorus.

More particularly, the term "heterocyclyl" is understood as being one of the rings (i) to (v) below:
- a 5-membered ring described by formula (i): 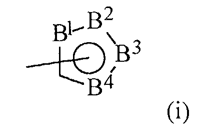 in which each of the groups of the list B¹, B², B³, B⁴ is chosen from carbon, nitrogen, oxygen and sulphur atoms such that the said list comprises from 0 to 3 carbon atoms, from 0 to 1 sulphur atom, from 0 to 1 oxygen atom and from 0 to 4 nitrogen atoms;
- a 6-membered ring described by formula (ii): 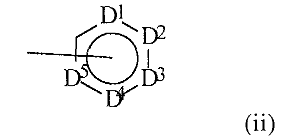 in which each of the groups of the list D¹, D², D³, D⁴, D⁵ is chosen from carbon and nitrogen atoms such that the said list comprises from 1 to 4 carbon atoms and from 1 to 4 nitrogen atoms;
- two fused rings, each being 6-membered, described by formula (iii): 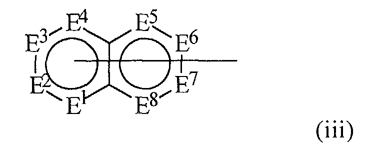 in which each of the groups in the list E¹, E², E³, E⁴, E⁵, E⁶, E⁷, E⁸ is chosen from carbon and nitrogen atoms such that the said list comprises from 4 to 7 carbon atoms and from 1 to 4 nitrogen atoms;
- a 6-membered ring and a 5-membered ring fused together, described by formula (iv): 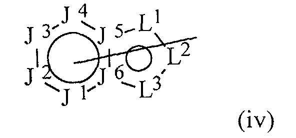 in which:
   * each of the groups in the list J¹, J², J³, J⁴, J⁵, J⁶ is chosen from carbon and nitrogen atoms such that the said list comprises from 3 to 6 carbon atoms and from 0 to 3 nitrogen atoms; and
   * each of the groups in the list L¹, L², L³ is chosen from carbon, nitrogen, oxygen and sulphur atoms such that the said list comprises from 0 to 3 carbon atoms, from 0 to 1 sulphur atom, from 0 to 1 oxygen atom and from 0 to 3 nitrogen atoms; and
   * each of the groups in the list J¹, J², J³, J⁴, J⁵, J⁶, L¹, L², L³ is chosen such that the said list comprises from 3 to 8 carbon atoms;
- two fused rings, each being 5-membered, described by formula (v): 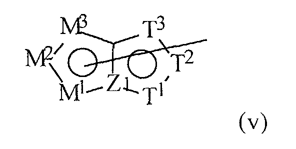 in which:
   each of the groups in the list M¹, M², M³ represents, carbon, nitrogen, oxygen or sulphur atoms such that the said list comprises from 0 to 3 carbon atoms, from 0 to 1 sulphur atom, from 0 to 1 oxygen atom and from 0 to 3 nitrogen atoms;
   each of the groups in the list T¹, T², T³ represents carbon, nitrogen, oxygen or sulphur atoms such that the said list comprises from 0 to 3 carbon atoms, from 0 to 1 sulphur atom, from 0 to 1 oxygen atom and from 0 to 3 nitrogen atoms;
   Z¹ represents a carbon or nitrogen atom;

   each of the groups in the list M¹, M², M³, T¹, T², T³ is chosen such that the said list comprises from 0 to 6 carbon atoms.

In the present invention, the term "heterocyclyl" even more particularly means: furyl, pyrolyl, thienyl, pyrazolyl, imidazolyl, oxazolyl, isoxazolyl, thiazolyl, isothiazolyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, 1,2,3-triazolyl, 1,2,4-triazolyl, tetrazolyl, pyridyl, pyrimidinyl, pyrazinyl, pyridazinyl, 1,2,3-triazinyl, 1,2,4-triazinyl, 1,3,5-triazinyl, 1,2,3,4-tetrazinyl, 1,2,3,5-tetrazinyl, 1,2,4,5-tetrazinyl, benzimidazolyl, indazolyl, benzotriazolyl, benzoxazolyl, 1,2-benzisoxazolyl, 2,1-benzisoxazolyl, benzothiazolyl, 1,2-benzisothiazolyl, 2,1-benzisothiazolyl, 1,2,3-benzoxadiazolyl, 1,2,5-benzoxadiazolyl, 1,2,3-benzothiadiazolyl, 1,2,5-benzothiadiazolyl, quinolyl, isoquinolyl, quinoxazolinyl, quinazolinyl, cinnolyl or phthalazyl, pteridinyl, benzotriazinyl, 1,5-naphthyridinyl, 1,6-naphthyridinyl, 1,7-naphthyridinyl, 1,8-naphthyridinyl, imidazo[2,1-b]thiazolyl, thieno[3,4-b]pyridyl, purine or pyrrolo[1,2-b]thiazolyl.

The compounds of general formula (I) and the compounds which may be used as intermediates in the processes of preparation, and which will be defined in the description of these processes, can exist in one or more forms of geometrical isomers according to the number of double bonds in the compound. The compounds of general formula (I) where Q, is -NR₁ or -N-NR₄R₅ can comprise 2 different geometrical isomers denoted (*E*) or (*Z*) depending on the configuration of the two double bonds. The *E* and *Z* notation can be replaced, respectively, by the term "syn" and "anti", or "cis" and "trans". Reference is made particularly to the work of E. Eliel and S. Wilen "Stereochemistry of Organic Compounds", published by Wiley (1994), for the description and use of these notations.

The compounds of general formula (I) and compounds which may be used as intermediates in the processes of preparation, and which will be defined in the description of the processes, can exist in one or more optical isomeric or chiral forms according to the number of asymmetric centres in the compound. The invention thus also relates to all the optical isomers and their racemic or scalemic (scalemic designates a mixture of enantiomers in different proportions), as well as the mixtures of all possible stereoisomers in all proportions, including the racemic mixture. The separation of the diastereoisomers and/or optical isomers can be effected by known methods (E. Eliel *ibid*.).

The present invention also relates to the process of preparation of the compounds of general formula (I) and compounds useful as intermediates in the processes of preparation. The compounds can be prepared according to the general method of preparation described below. Although general, this method of preparation provides all of the operating conditions to be used for the synthesis of the compounds of formula (I) according to the present invention. It will nevertheless be understood that the skilled worker will be able to adapt this method according to the specifics of each of the compounds, which it is desired to synthesise.

The preparation of reagents used in one or other of the general methods of preparation is generally known and is generally described specifically in the prior art or in such a manner that the man skilled in the art can adapt it to the desired aim. The prior art usable by the normally skilled worker in order to establish conditions for the preparation of reagents can be found in numerous general chemistry text books such as "Advanced Organic Chemistry" by J. March, published by Wiley (1992), "Methoden der organischen Chemie" (Houben-Weyl), published by Georg Thieme Verlag or the "Chemical Abstracts" published by the American Chemical Society as well as in information data bases accessible to the public.

In the following description, it has to be understood that compounds of formula (I-A) are compounds of formula (I) in which Y and G, together with carbon 3 and 4 form ring A, as defined above; that compounds of formula (I-B) are compounds of formula (I) in which Y and G, together with carbon 3 and 4 form ring B, as defined above; etc. Compounds (I-A) to (I-O) all together form compounds of formula (I) as defined above.

Compounds of general formula (I-A to I-O), in which n is equal to 0, may be prepared from a compound of formula (II) (prepared according to the method described in the non published international patent application no. PCT/FR01/00033): 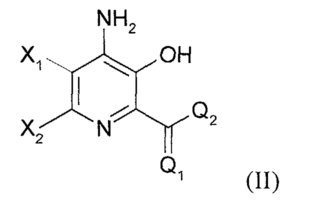 in which X₁, X₂, Q₁ and Q₂ are as defined above.

Compounds of general formula (I-A to I-O), in which n is equal to 1, may be prepared from corresponding compounds of formulae (I-A) to (I-O) respectively in which n is equal to 0 by pyridine oxidation according to general methods known by the skilled artisan.

The following processes show how to prepare compounds of formulae (I-A). (I-B) and (I-C). From these general descriptions, the man skilled in the art will be able to aesily prepare the other compounds of formulae (I-D) to (I-O) from the data given in the description and from his general knowledge of organic chemistry.

The compound (II) is placed in contact with a phosgene or thiophosgene solution, this reaction preferably being carried out in an apolar aprotic solvent such as benzene or toluene, at reflux or at a temperature of between 20°C and 200°C, to give the compounds of formula (I-A): 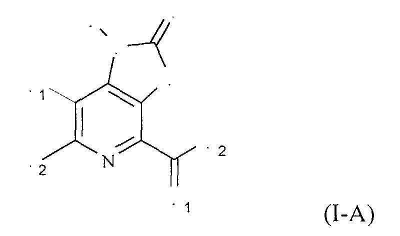 in which J, X, W, X₁, X₂, Q₁ and Q₂ are as defined above.

The compound (II) is placed in contact with acid chloride, this reaction preferably being carried out in an apolar aprotic solvent such as benzene or toluene, at reflux or at a temperature of between 20°C and 200°C, to give the compounds of formula (I-B): 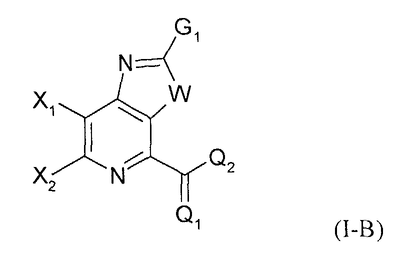 in which G₁, X, W, X₁, X₂, Q₁ and Q₂ are as defined above.

The compound (II) is placed in contact with alkyl or aryl ketone, this reaction preferably being carried out in an apolar aprotic solvent such as benzene or toluene, at reflux or at a temperature of between 20°C and 200°C, to give the compounds of formula (I-C): 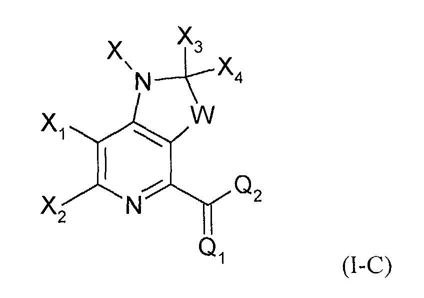 in which X, W, X₁, X₂, X₃, X₄, Q₁ and Q₂ are as defined above.

It should be understood that the reactions described in the preceding paragraphs may be carried out in any other order, which is suitable to obtain the desired compounds of formula (I). The order of the reactions will be determined most particularly by the compatibility requirements of the various substituants on the pyridine nucleus. Compatibilities of the various radicals and reagents used are well known to the person skilled in the art, who may moreover refer to the examples for the preparation of the compounds of formula (I) described later in this description.

The invention also relates to fungicidal compositions comprising an effective amount of at least one active material of formula (I). The fungicidal compositions according to the invention generally comprise, besides the active material of formula (I), agriculturally acceptable solid or liquid supports and/or surfactants that are also agriculturally acceptable. In particular, common inert supports and common surfactants can be used. These compositions include not only compositions which are ready to be applied to the plant or seed to be treated by means of a suitable device. such as a spraying or dusting device, but also concentrated commercial compositions which must be diluted before they are applied to the crop.

These fungicidal compositions according to the invention can also contain other ingredients of any kind, such as, for example, protective colloids, adhesives, thickeners, thixotropic agents, penetration agents, stabilisers, sequestering agents, etc. More generally, the active materials can be combined with any solid or liquid additive, which complies with the usual formulation techniques.

In general, the compositions according to the invention usually contain from 0.05 to 99% (by weight) of active material, one or more solid or liquid supports and, optionally, one or more surfactants.

In the present specification, the term "support" denotes a natural or synthetic, organic or inorganic material with which the active material is combined to make it easier to apply to the parts of the plant. This support is thus generally inert and should be agriculturally acceptable. The support can be solid (clays, natural or synthetic silicates, silica, resins, waxes, solid fertilisers, etc.) or liquid (water. alcohols, in particular butanol, etc., organic solvents, mineral and plant oils and derivatives thereof). Mixtures of such supports may also be used.

The surfactant can be an emulsifier, a dispersing agent or a wetting agent of ionic or non-ionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the above compounds containing sulphate, sulphonate and phosphate functions. The presence of at least one surfactant is generally essential when the active material and/or the inert support are water-insoluble and when the vector agent for the application is water.

Thus, the compositions for agricultural use according to the invention can contain the active material in a very wide range, from 0.05% to 99% (by weight). Their surfactant content is advantageously between 5% and 40% by weight. Except where otherwise indicated, the percentages given in this description are weight percentages.

These compositions according to the invention are themselves in quite diverse, solid or liquid forms. As solid composition forms, mention may be made of powders for dusting (with an active material content which can be up to 100%) and granules, in particular those obtained by extrusion, by atomisation, by compacting, by impregnation of a granulated support or by granulation from a powder (the active material content in these granules being between 0.5 and 80% for the latter eases).

The fungicidal compositions according to the invention can also be used in the form of powders for dusting; compositions comprising 50 g of active material and 950 g of talc can also be used; compositions comprising 20 g of active material, 10 g of finely divided silica and 970 g of talc can also be used; these constituents are mixed together and ground and the mixture is applied by dusting.

As liquid composition forms or forms intended to constitute liquid compositions when applied, mention may be made of solutions, in particular water-soluble concentrates, emulsifiable concentrates, emulsions, concentrated suspensions and wettable powders (or powders for spraying).

The concentrated suspensions, which can be applied by spraying, are prepared so as to obtain a stable fluid product which does not become deposited and which gives good bioavailability of the active material(s). These suspensions usually contain from 5% to 75% of active material, preferably from 10% to 25%, from 0.5% to 75% of surfactants, preferably from 5% to 50%, from 0% to 10% of suitable additives, such as thickeners of organic or mineral origin, antifoaming agents, corrosion inhibitors, adhesives, preserving agents, such as, for example, Proxel GXL®, antifreezes and, as support, water or an organic liquid in which the active material is insoluble or only sparingly soluble: certain organic solid materials or mineral salts may be dissolved in the support to help prevent sedimentation or as antifreezes for the water. In certain cases, and in particular for formulations intended for treating seeds, one or more colorants may be added.

For foliar applications, the choice of surfactants is paramount for obtaining good bioavailability of the active material(s); thus, a combination of a surfactant of hydrophilic nature (HLB>10) and a surfactant of lipophilic nature (HLB<5) will preferably be used. Such combinations of surfactants are disclosed, for example, in the as yet unpublished French patent No. 00/04015.

By way of example, here are 3 possible formulations of concentrated suspension type that are suitable for various crops:

### Example CS 1 (in k/kg):

This example is rather suited for monocotyledon crops (cereals, rice, etc.)
- active material 150
- surfactant of hydrophilic nature (for example Rhodasurf 860P) 300
- surfactant of lipophilic nature (for example Plurafac LF 700) 150
- ethoxylated tristyrylphenol phosphate 50
- antifoam 5
- propylene glycol 30
- Aerosil 200 20
- Attagel 50 40
- water (completion to 1 kg) 255

### Example CS 2 (in g/kg):

This example is suited rather to dicotyledon crops (vines, fruit trees, etc.)
- active material 150
- surfactant of hydrophilic nature (for example Rhodasurf 860P) 150
- ethoxylated tristyrylphenol phosphate 50
- antifoam 5
- propylene glycol 30
- Aerosil 200 20
- Attagel 50 40
- water (completion to 1 kg) 555

### Example CS 3 (in k/kg):

This example is more specifically suited to the treatment of seeds.
- active material 50
- surfactant of hydrophilic nature (for example Rhodasurf 860P) 5
- ethoxylated tristyrylphenol phosphate 15
- antifoam 1
- propylene glycol 30
- colorant 20
- Rhodopol G 1.5
- Proxel GXL 1.5
- water (completion to 1 kg) 876

The following procedure will preferably be carried out to prepare these formulations: The surfactants selected (surfactant of hydrophilic nature + surfactant of lipophilic nature + ethoxylated tristyrylphenol phosphate) are mixed with the required amount of water, using a turbo mixer; after homogenization, the other constituents of the formulation apart from the active material are then mixed together.

Next, the active material and optionally the thickener of mineral origin (Aerosol 200 and Attagel 50) are added to give a medium of viscous consistency.

The mixture obtained is then ground using a turbo mixer mill at high speed and then a ball mill until a D50 of about 1 to 3 µm and a D90 of between 3 and 8 µm are obtained.

When no thickener of mineral origin is used, the thickener of natural origin (Rhodopol G) is then added and the mixture is stirred until a suitable viscosity is obtained.

The wettable powders (or powders for spraying) are usually prepared such that they contain 20 to 95% of active material, and they usually contain, in addition to the solid support, from 0 to 30% of a wetting agent, from 3 to 20% of a dispersant and. when necessary, from 0.1 to 10% of one or more stabilisers and/or other additives, such as penetration agents, adhesives, anticaking agents, dyes, etc.

In order to obtain the powders for spraying or wettable powders, the active materials are intimately mixed with the additional substances in suitable mixers and are ground with mills or other suitable blenders. Powders for spraying with advantageous wettability and suspension formation are thus obtained; they can be placed in suspension with water at any desired concentration and these suspensions can be used very advantageously, in particular for application, for example, to plant leaves or to seeds.

By way of example, there follow various wettable powder compositions (or powders for spraying):

### Example WP1

- Active material 50%
- Ethoxylated fatty alcohol (wetting agent) 2.5%
- Ethoxylated phenylethylphenol (dispersant) 5%
- Chalk (inert support) 42.5%

### Example WP2:

- Active material 10%
- Synthetic C13 oxo alcohol of branched type, ethoxylated with 8 to 10 ethylene oxide (wetting agent) 0.75%
- Neutral calcium lignosulphonate (dispersant) 12%
- Calcium carbonate (inert filler) completion to 100%

### Example WP3:

This wettable powder contains the same ingredients as in the above example, in the following proportions:
- Active material 75%
- Wetting agent 1.50%
- Dispersant 8%
- Calcium carbonate (inert filler) completion to 100%

### Example WP4:

- Active material 90%
- Ethoxylated fatty alcohol (wetting agent) 4%
- Ethoxylated phenylethylphenol (dispersant) 6%

### Example WP5:

- Active material 50%
- Mixture of anionic and non-ionic surfactants (wetting agent) 2.5%
- Sodium lignosulphonate (dispersant) 5%
- Kaolinic clay (inert support) 42.5%

The aqueous dispersions and emulsions, for example the compositions obtained by diluting a wettable powder according to the invention with water, are included within the general scope of the present invention. The emulsions can be of the water-in-oil or oil-in-water type and they can have a thick consistency, such as that of a "mayonnaise".

The fungicidal compositions according to the invention can be formulated in the form of water-dispersible granules, which are also included within the scope of the invention. These dispersible granules, with an apparent density generally of between about 0.3 and about 0.6, have a particle size generally of between about 150 and about 2000 and preferably between 300 and 1500 microns.

The active material content of these granules is generally between about 1% and about 90% and preferably between 25% and 90%. The rest of the granule is essentially composed of a solid support and optionally of surfactant adjuvants, which give the granule water-dispersibility properties. These granules can be essentially of two different types depending on whether the selected support is soluble or insoluble in water. When the support is water-soluble, it can be inorganic or, preferably, organic. Excellent results have been obtained with urea. In the case of an insoluble support, it is preferably inorganic, for example such as kaolin or bentonite. It is then advantageously accompanied by surfactants (in a proportion of from 2 to 20% by weight of the granule) more than half of which consists, for example, of at least one dispersant, which is essentially anionic, such as an alkali metal or alkaline-earth metal polynaphthalene sulphonate or an alkali metal or alkaline-earth metal lignosulphonate, the remainder consisting of non-ionic or anionic wetting agents such as an alkali metal or alkaline-earth metal alkylnaphthalene sulphonate. Moreover, although this is not essential, other adjuvants can be added, such as antifoaming agents.

The granule according to the invention can be prepared by mixing together the required ingredients, followed by granulation according to several techniques which are known per se (granulator, fluid bed, sprayer, extrusion, etc.). The process generally ends by a crushing operation, followed by an operation of screening to the particle size chosen within the limits mentioned above. Granules obtained as above and then impregnated with a composition containing the active material can also be used.

Preferably, it is obtained by extrusion, by performing the process as indicated in the examples below.

### Example DG1: Dispersible granules

90% by weight of active material and 10% of urea pellets are mixed together in a mixer. The mixture is then ground in a toothed roll crusher. A powder is obtained. which is moistened with about 8% by weight of water. The wet powder is extruded in a perforated-roller extruder. A granulate is obtained, which is dried and then crushed and screened, so as to retain, respectively, only the granules between 150 and 2000 microns in size.

### Example DG2: Dispersible granules

The constituents below are mixed together in a mixer:
- Active material 75%
- Wetting agent (sodium alkylnaphthalene sulphonate) 2%
- Dispersant (polysodium naphthalene sulphonate) 8%
- Water-insoluble inert filler (kaolin) 15%

This mixture is granulated in a fluid bed, in the presence of water, and then dried, crushed and screened so as to obtain granules between 0.15 and 0.80 mm in size.

These granules can be used alone, or as a solution or dispersion in water so as to obtain the desired dose. They can also be used to prepare combinations with other active materials, in particular fungicides, these being in the form of wettable powders, granules or aqueous suspensions.

The compounds of the invention can also be mixed with one or more insecticides, fungicides, bactericides, attractant acaricides or pheromones or other compounds with biological activity. The mixtures thus obtained have a broadened spectrum of activity. In particular the compounds of the present invention do not exhibit the problem of cross-resistance with strobilurin derivatives. In fact the compounds of the present invention are active on a different biochemical site to strobilurin derivatives.

The mixtures with other fungicides are particularly advantageous, especially the mixtures with acibenzolar-S-methyl, benalaxyl, benomyl, blasticidin-S, bromuconazole, captafol, captan, carbendazim, carboxin, carpropamide, chlorothalonil, fungicidal compositions based on copper, derivatives of copper such as copper hydroxide and copper oxychloride, cyazofamide, cymoxanil, cyproconazole, cyprodinil, dichloran, diclocymet, diethofencarb, difenoconazole, diflumetorim, dimethomorph, diniconazole, dodemorph, dodine, edifenphos, epoxyconazole, ethaboxam, ethirimol, famoxadone, fenamidone, fenarimol, fenbuconazole, fenhexamide, fenpiclonil, fenpropidine, fenpropimorph, ferimzone, fluazinam, fludioxonil, flumetover, fluquinconazole, flusilazole, flusulfamide, flutolanil, flutriafol, folpel, furalaxyl, furametpyr, guazatine, hexaconazole, hymexazol, imazalil, iprobenphos, iprodione, isoprothiolane, kasugamycin, mancozeb, maneb, mefenoxam, mepanipyrim, metalaxyl and their enantiomeric forms such as metalaxyl-M, metconazole, metiram-zinc, oxadixyl, pefurazoate, penconazole, pencycuron, phosphorous acid and its derivatives such as fosetyl-Al, phthalide, probenazole, prochloraz, procymidone, propamocarb, propiconazole, pyrimethanil, pyroquilon, quinoxyfen, silthiofam, simeconazole, spiroxamine, tebuconazole, tetraconazole, thiabendazole, thifluzamide, thiophanate, for example thiophanate-methyl, thiram, triadimefon, triadimenol, triazolopyrimidines e.g. cloransulam-methyl, flumetsulam, florasulam, metosulam, tricyclazole, tridemorph, trifloxystrobin, triticonazole, derivatives of valinamide such as for example, iprovalicarb and benthiavalicarb, vinclozolin, zineb and zoxamide, as well as fungicide of the strobilurin familly, for example azoxystrobin, kresoxym-methyl, metominostrobin, discostrobin, dimoxystrobin, picoxystrobin, pyraclostrobin, and trifloxystrobin.

Another subject of the invention is a process for curatively or preventively combating the phytopathogenic fungi of crops, characterised in that an effective (agronomically effective) and non-phytotoxic amount of an active material of formula (I), preferably in the form of a fungicidal composition according to the invention, is applied to the plant seeds or to the plant leaves and/or to the fruits of the plants or to the soil in which the plants are growing or in which it is desired to grow them.

The expression "effective and non-phytotoxic amount" means an amount of composition according to the invention which is sufficient to control or destroy the fungi present or liable to appear on the crops, and which does not entail any appreciable symptom of phytotoxicity for the said crops. Such an amount can vary within a wide range depending on the fungus to be combated, the type of crop, the climatic conditions and the compounds included in the fungicidal composition according to the invention.

This amount can be determined by systematic field trials, which are within the capabilities of a person skilled in the art.

Lastly, the invention relates to a process for preventively or curatively protecting plant multiplication products, as well as the plants resulting therefrom, against fungal diseases, characterised in that the said products are coated with an effective and non-phytotoxic dose of a composition according to the invention.

The compositions according to the invention are useful for treating the seeds of cereals (wheat, rye, triticale and barley in particular), of potato, of cotton, of pea, of rape, of corn or of flax, or the seeds of woodland trees (in particular resiniferous trees) or genetically modified seeds of these plants.

It will be noted in this respect that, in the jargon of the person skilled in the art, the expression "seed treatment" in fact refers to treatment of the plant multiplication products. The application techniques are well known to those skilled in the art and they may be used without a drawback in the context of the present invention. Mention may be made, for example, of film cladding or coating. Among the plant multiplication products concerned, mention may be made in particular of seeds or grains, and tubers.

As has been indicated above, the methods for coating the plant multiplication products, in particular the seeds, are well known in the art and in particular involve film-cladding or coating techniques.

The products and compositions according to the invention may also be applied as a foliar application to the plant crops, i.e. to the leaves, the flowers, the fruit and/or the trunks of the plants concerned.

Among the plants targeted by the method according to the invention, mention may be made of rice, corn, cotton, cereals, for instance wheat, barley, triticale, fruit trees, in particular apple trees, pear trees, peach trees, vines, banana trees, orange trees, lemon trees, etc., oil-yielding crops, for example rape and sunflower, market-garden crops and leguminous crops, tomatoes, lettuce, protein-yielding crops, peas, solanaceous plants, for example potato, beetroot and flax, and woodland trees, as well as genetically modified homologues of these crops.

Among the plants targeted by the method according to the invention, mention may be made of:
- wheat, as regards controlling the following seed diseases: fusaria (*Microdochium nivale* and *Fusarium roseum*), stinking smut (*Tilletia caries*, *Tilletia controversa* or *Tilletia indica*), septoria disease (*Septoria nodorum*) and loose smut;
- wheat, as regards controlling the following diseases of the aerial parts of the plant: cereal eyespot (*Tapesia yallundae, Tapesia acuiformis*), take-all (*Gaeumannomyces graminis*), foot blight (*F. culmorum*, *F. graminearum*), black speck (*Rhizoctonia cerealis*), powdery mildew (*Erysiphe graminis forma specie tritici*), rusts (*Puccinia striiformis* and *Puccinia recondita*) and septoria diseases (*Septoria tritici* and *Septoria nodorum*);
- wheat and barley, as regards controlling bacterial and viral diseases, for example barley yellow mosaic;
- barley, as regards controlling the following seed diseases: net blotch (*Pyrenophora graminea*, *Pyrenophora teres* and *Cochliobolus sativus*), loose smut (*Ustilago nuda*) and fusaria (*Microdochium nivale* and *Fusarium roseum*);
- barley, as regards controlling the following diseases of the aerial parts of the plant: cereal eyespot (*Tapesia yallundae*), net blotch (*Pyrenophora teres* and *Cochliobolus sativus*), powdery mildew (*Erysiphe graminis forma specie hordei*), dwarf leaf rust (*Puccinia hordei*) and leaf blotch (*Rhynchosporium secalis*);
- potato, as regards controlling tuber diseases (in particular *Helminthosporium solani, Phoma tuberosa, Rhizoctonia solani, Fusarium solani*), mildew (*Phytopthora infestans*) and certain viruses (virus Y);
- potato, as regards controlling the following foliage diseases: early blight (*Alternaria solani*), mildew (*Phytophthora infestans*);
- cotton, as regards controlling the following diseases of young plants grown from seeds: damping-off and collar rot (*Rhizoctonia solani, Fusarium oxysporum*) and black root rot (*Thielaviopsis basicola*);
- protein yielding crops, for example peas, as regards controlling the following seed diseases: anthracnose (*Ascochyta pisi, Mycosphaerella pinodes*), fusaria (*Fusarium oxysporum*), grey mould (*Botrytis cinerea*) and mildew (*Peronospora pisi*);
- oil-bearing crops, for example rape, as regards controlling the following seed diseases: *Phoma lingam, Alternaria brassicae* and *Sclerotinia sclerotiorum*;
- corn, as regards controlling seed diseases: (*Rhizopus* sp., *Penicillium* sp., *Trichoderma* sp., *Aspergillus* sp., and *Gibberella fujikuroi*);
- flax, as regards controlling the seed disease: *Alternaria linicola*;
- forest trees, as regards controlling damping-off (*Fusarium oxysporum, Rhizoctonia solani*);
- rice, as regards controlling the following diseases of the aerial parts: blast disease (*Magnaporthe grisea*), bordered sheath spot (*Rhizoctonia solani*);
- leguminous crops, as regards controlling the following diseases of seeds or of young plants grown from seeds: damping-off and collar rot (*Fusarium oxysporum, Fusarium roseum*, *Rhizoctonia solani*, *Pythium sp.*);
- leguminous crops, as regards controlling the following diseases of the aerial parts: grey mould (Botrytis sp.), powdery mildews (in particular *Erysiphe* *cichoracearum, Sphaerotheca fuliginea* and *Leveillula taurica*), fusaria (*Fusarium oxysporum, Fusarium roseum*), leaf spot (*Cladosporium sp*.), alternaria leaf spot (*Alternaria sp*.), anthracnose (*Colletotrichum sp*.), septoria leaf spot (*Septoria sp*.), black speck (*Rhizoctonia solani*), mildews (for example *Bremia lactucae, Peronospora sp., Pseudoperonospora sp.*, *Phytophthora sp*.);
- fruit trees, as regards diseases of the aerial parts: monilia disease (*Monilia fructigenae, M. laxa*), scab (*Venturia inaequalis*), powdery mildew (*Podosphaera leucotricha*);
- vine, as regards diseases of the foliage: in particular grey mould (*Botrytis cinerea*), powdery mildew (*Uncinula necator*), black rot (*Guignardia biwelli*) and mildew (*Plasmopara viticola*);
- beetroot, as regards the following diseases of the aerial parts: cercospora blight (*Cercospora beticola*), powdery mildew (*Erysiphe beticola*), leaf spot (*Ramularia beticola*).

Wheat and rice are the preferred plants for carrying out the method according to the invention, although all the crops, plants, plant multiplication products, flowers, wood and, as a general rule, all the plants which may suffer attack by phytopathogenic fungi, may be advantageously treated according to the method of the invention, by using one or more active materials, fungicidal compositions according to the present invention.

In the case of plant treatments, the dose of composition applied is generally and advantageously between 10 and 800 g/ha, preferably 50 to 300 g/ha of active material for applications in foliar treatment. The dose of composition applied is generally and advantageously such that the dose of active material is between 2 and 200 g of active material per 100 kg of seed, preferably between 3 and 150 g per 100 kg in the case of seed treatments. It is clearly understood that the doses indicated above are given as illustrative examples of the invention. A person skilled in the art will know how to tailor the application doses according to the nature of the crop to be treated.

The present invention also relates to a method for curatively or preventively treating, with the aid of one or more compounds according to the invention, or of a composition according to the present invention, against fungal diseases liable to grow on or inside lumber. The term "lumber" means all types of species of wood, and all types of working of this wood intended for construction, for example solid wood, high-density wood, laminated wood, plywood, etc.

Thus, the method for treating lumber according to the invention consists in placing one or more compounds of the present invention, or a composition according to the invention, in contact. This placing in contact may cover the most diverse of forms such as, for example, direct application, spraying, dipping, injection or any other suitable means.

The present invention also relates to the treatment of genetically modified plants with the compounds according to the invention or the agrochemical compositions according to the invention. Genetically modified plants are plants into whose genome a heterologous gene encoding a protein of interest has been stably integrated.

According to the invention, the expression "heterologous gene encoding a protein of interest" essentially means genes which give the transformed plant new agronomic properties, or genes for improving the agronomic quality of the transformed plant.

Among the genes which give the transformed plants new agronomic properties, mention may be made of genes which impart a tolerance to certain herbicides, those which impart a resistance to certain insects, those which impart a tolerance to certain diseases, etc. Such genes are described in particular in patent applications WO 91/02071 and WO 95/06128.

Among the genes which impart a tolerance to certain herbicides, mention may be made of the *Bar* gene imparting tolerance to bialophos, the gene encoding a suitable EPSPS imparting a resistance to herbicides having EPSPS as target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5,633,435 and FR 2 736 926), the gene encoding glyphosate oxidoreductase (US 5 463 175) or a gene encoding an HPPD imparting a tolerance to herbicides having HPPD as target, such as isoxazoles, in particular isoxafutol (FR 95/06800 and FR 95/13570), diketonitriles (EP-A-0 496 630 and EP-A-0 496 631) or triketones, in particular sulcotrioine (EP-A-0 625 505, EP-A-0 625 508 and US 5 506 195). Such genes encoding an HPPD imparting a tolerance to herbicides having HPPD as target are disclosed in patent application WO 96/38567.

In the case of genes encoding EPSPS or HPPD, and more particularly for the above genes, the sequence encoding these enzymes is advantageously preceded by a sequence encoding a transit peptide, in particular for the transit peptide known as optimized transit peptide, disclosed in patent US 5 510 471.

Among the genes imparting novel insect-resistance properties, mention will be made more particularly of the genes encoding the *Bt* proteins which are widely described in the literature and well known to those skilled in the art. Mention will also be made of the genes encoding proteins extracted from bacteria such as *Photorabdus* (WO 97/17432 and WO 98/08932).

Among the genes imparting novel disease-resistance properties, mention will be made in particular of the genes encoding chitinases, glucanases and oxalate oxidase, all these proteins and their coding sequences being widely described in the literature, or genes encoding antibacterial and/or antifungal peptides, in particular cysteine-rich peptides containing less than 100 amino acids, such as plant thionines or defensines, and more particularly lytic peptides of all origins comprising one or more disulphide bridges between the cysteines and regions comprising basic amino acids, in particular the following lytic peptides: androctonine (WO 97/30082 and PCT/FR98/01814, filed on 18 August 1998) or drosomicin (PCT/FR98/01462, filed on 8 July 1998). Mention will also be made of the genes encoding fungal elicitor peptides, in particular the elicitins (Kamoun et al., 1993; Panabières et al., 1995).

Among the genes which modify the constitution of modified plants, mention may be made in particular of genes which modify the content and quality of certain essential fatty acids (EP-A-0 666 918) or the content and quality of proteins, in particular in the leaves and/or seeds of the said plants. Mention will be made in particular of the genes encoding proteins that are rich in sulphur-containing amino acids (WO 98/20133; WO 97/41239; WO 95/31554; WO 94/20828 and WO 92/14822).

The present invention relates more particularly to the treatment of genetically modified plants comprising a heterologous gene, which gives the plant disease-resistance properties. The heterologous gene preferentially gives the genetically modified plant a spectrum of activity that is complementary to the spectrum of activity of the compounds according to the invention. According to the invention, the expression "complementary spectrum" means a spectrum of activity for the heterologous gene which is different from the spectrum of activity of the compounds according to the invention, or a spectrum of activity relating to identical infectious agents but allowing an identical or improved control for lower application doses of compounds according to the invention.

Lastly, the invention relates to the use of the compounds according to the invention that are useful in human and animal therapy for the treatment of fungal diseases such as, for example, mycoses, dermatoses, trichophyton diseases and candidiases or diseases caused by *Aspergillus spp*., for example *Aspergillus fumigatus.*

The following Examples (Table 1) illustrate in a non-limiting manner several examples of fungicidal compounds according to the invention. In the following Examples, "MP" signifies "melting point" and is expressed in ° Celsius (°C). 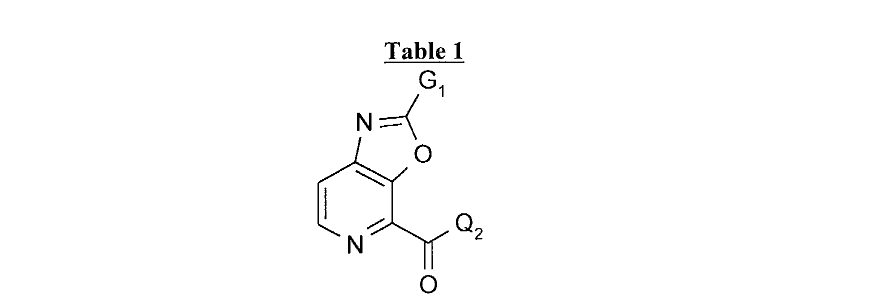 As an example, compound No. 3 has been prepared according to the following

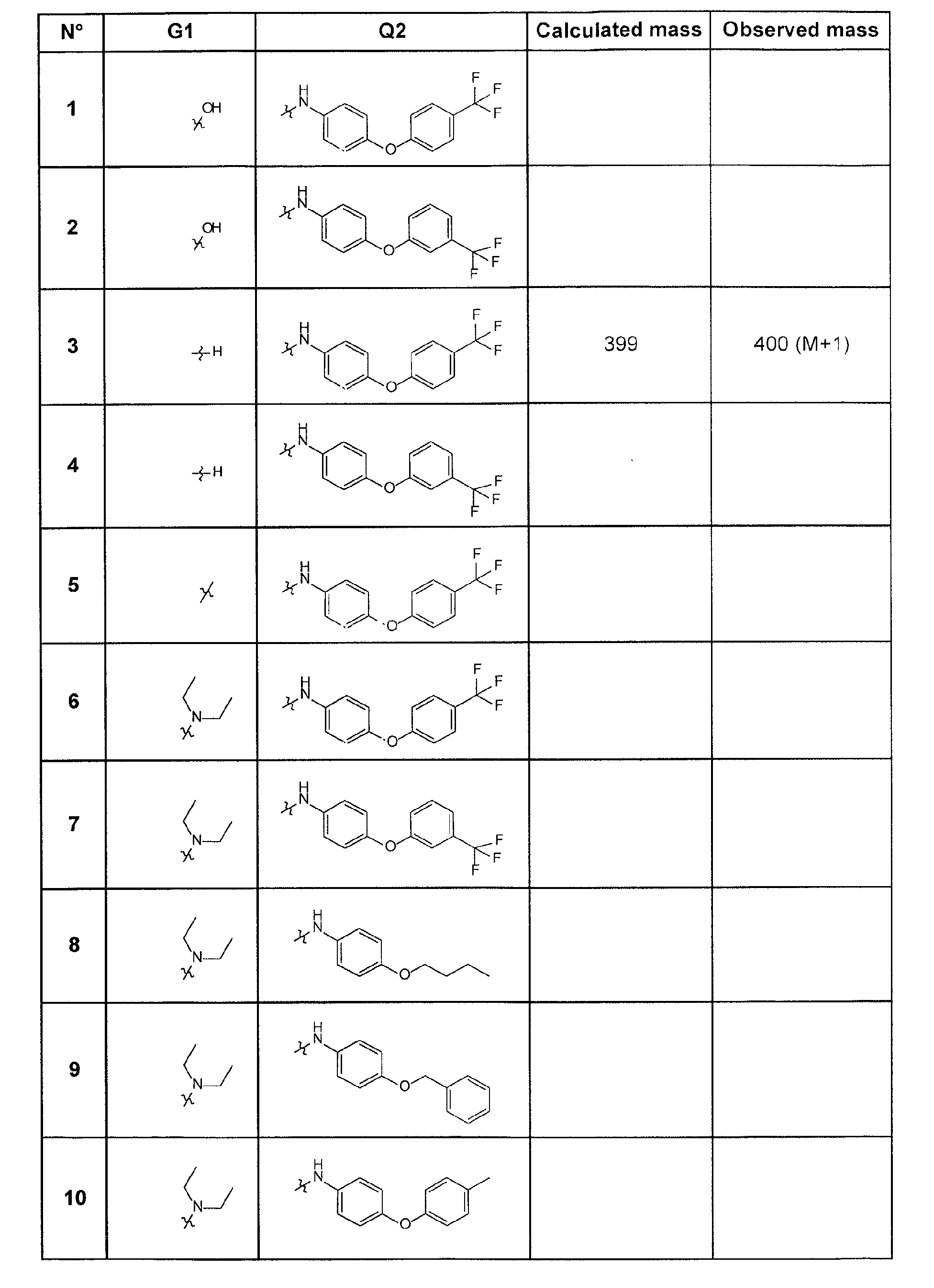

As an example, compound No. 3 has been prepared according to the following process:

### Example 1: Preparation of compound no. 3

4-Amino-3-hydroxy-N-*para*-[4-(trifluoromethyl)phenoxy]phenylpicolinamide (1.3 g, 3.3 mmol; prepared according to PCT/FR01/00033) in 20 mL of triethyl orthoformate with a catalytic amount of *p*-toluene sulphonic acid are heated under reflux for 3 hours. After cooling, the triethyl orthoformate was evaporated and the residue chromatography on silica gel. There is obtained 250 mg of the title compound, as a yellow solid (APCI+, 400, M+1).

### Examples of Biological Activity of the Compounds of the Invention

### Example A: in vivo test on Alternaria brassicae (Leaf spot of crucifers):

An aqueous suspension, at a concentration of 1.5%, of the active material tested is obtained by grinding it finely in a formulation of concentrated suspension type. This aqueous suspension is then diluted in water so as to obtain the desired active material concentration. Radish plants (Pernot variety) in starter cups, sown on a 50/50 peat soil-pozzolana substrate and grown at 18-20°C, are treated at the cotyledon stage by spraying with the aqueous suspension described above. Plants, used as controls, are treated with an aqueous solution not containing the active material. After 24 hours, the plants are contaminated by spraying them with an aqueous suspension of *Alternaria brassicae* spores (40,000 spores per cm³). The spores are collected from a 12-13 day-old culture. The contaminated radish plants are incubated for 6-7 days at about 18°C, under a humid atmosphere. Grading is carried out 6 to 7 days after the contamination, in comparison with the control plants. Under these conditions, good (at least 50%) or total protection is observed at a dose of 500 g/ha with a number of compounds of the present invention.

### Example B: in vivo test on Septoria nodorum (wheat Glume blotch):

An aqueous suspension, at a concentration of 1.5%, of the active material tested is obtained by grinding it finely in a formulation of concentrated suspension type. This aqueous suspension is then diluted in water so as to obtain the desired active material concentration.
Wheat plants (Scipion variety) in starter cups, sown on a 50/50 peat soil-pozzolana substrate and grown at 12°C, are treated at the 1-leaf stage (10 cm tall) by spraying them with the aqueous suspension described above. Plants, used as controls, are treated with an aqueous solution not containing the active material. After 24 hours, the plants are contaminated by spraying with an aqueous suspension of *Septoria nodorum* spores (500,000 spores per cm³). The spores are collected from a seven-day-old culture. The contaminated wheat plants are incubated for 72 hours at about 18°C, under a humid atmosphere, and then for 14 days at 90% relative humidity.
Grading is carried out 15 to 20 days after contamination, in comparison with the control plants. Under these conditions, good (at least 50%) or total protection is observed, at a dose of 500 g/ha, with a number of compounds of the present invention.

### Example C: in vivo test on Erisyphe graminis f. sp. tritici (wheat powdery mildew):

An aqueous suspension, at a concentration of 1.5%, of the active material tested is obtained by grinding it finely in a formulation of concentrated suspension type. This aqueous suspension is then diluted in water so as to obtain the desired active material concentration. Wheat plants (Audace variety) in starter cups, sown on 50/50 peat soil-pozzulana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by dusting them with *Erisyphe graminis* f. sp. *tritici* spores, the dusting being carried out using diseased plants.
Grading is carried out 7 to 14 days after the contamination, in comparison with the control plants. Under these conditions, good (at least 50%) or total protection is observed, at a dose of 500 g/ha, with a number of compounds of the present invention.

### Example D: in vivo test on Septoria tritici (Leaf spot of wheat):

An aqueous suspension, at a concentration of 1.5%, of the active material tested is obtained by grinding it finely in a formulation of concentrated suspension type. This aqueous suspension is then diluted in water so as to obtain the desired active material concentration. Wheat plants (Scipion variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by spraying them with an aqueous suspension of *Septoria tritici* spores (500,000 spores per mL). The spores are collected from a 15-day-old culture and are suspended in a nutrient solution composed of:
- 1.5 g/L of gelatin
- 0.5 g/L of sodium oleate
- 24 g/L of PDB

The contaminated wheat plants are incubated for 72 hours at about 20°C and at 100% relative humidity, and then for 15 days at 80% relative humidity.
Grading is carried out 15 to 20 days after the contamination, in comparison with the control plants. Under these conditions, good (at least 50%) protection is observed, at a dose of 500 g/ha, with a number of compounds of the present invention.

### Example E: in vivo test on Drechslera teres (Barley Net blotch):

An aqueous suspension, at a concentration of 1.5%, of the active material tested is obtained by grinding it finely in a formulation of concentrated suspension type. This aqueous suspension is then diluted in water so as to obtain the desired active material concentration. Barley plants (Express variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by spraying them with an aqueous suspension of *Dreschslera teres* spores (12,000 spores per mL). The spores are collected from a 12-day-old culture .The contaminated wheat plants are incubated for 24 hours at about 20°C and at 100% relative humidity, and then for 12 days at 80% relative humidity.
Grading is carried out 12 days after the contamination, in comparison with the control plants. Under these conditions, good (at least 50%) protection is observed, at a dose of 500 g/ha, with a number of compounds of the present invention.

### Example F: in vivo test on Rhynchosporium secalis (Barley leaf scald):

An aqueous suspension, at a concentration of 1.5%, of the active material tested is obtained by grinding it finely in a formulation of concentrated suspension type. This aqueous suspension is then diluted in water so as to obtain the desired active material concentration. Barley plants (Express or Barrack variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by spraying them with an aqueous suspension of *Rhynchosporium secalis* spores (800,000 spores per mL). The contaminated wheat plants are incubated for 48 hours at about 20°C and at 100% relative humidity, and then for 12/14 days at 80% relative humidity.
Grading is carried out 12/14 days after the contamination, in comparison with the control plants. Under these conditions, good (at least 50%) protection is observed, at a dose of 500 g/ha, with a number of compounds of the present invention.

### Example G: in vivo test on Puccinia recondita (Wheat brown rust):

An aqueous suspension, at a concentration of 1.5%, of the active material tested is obtained by grinding it finely in a formulation of concentrated suspension type. This aqueous suspension is then diluted in water so as to obtain the desired active material concentration. Wheat plants (Scipion variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by spraying them with an aqueous suspension of *Puccinia recondita* spores (150,000 spores per mL). The contaminated wheat plants are incubated for 24 hours at about 20°C and at 100% relative humidity, and then for 10 days at 70 % relative humidity.
Grading is carried out 10 days after the contamination, in comparison with the control plants. Under these conditions, good (at least 50%) protection is observed, at a dose of 500 g/ha, with a number of compounds of the present invention.

### Example H: in vivo test on Botrytis cinerea (cucumber Grey mould):

An aqueous suspension, at a concentration of 1.5%, of the active material tested is obtained by grinding it finely in a formulation of concentrated suspension type. This aqueous suspension is then diluted in water so as to obtain the desired active material concentration. Cucumber plants (Marketer variety) in starter cups, sown on a 50/50 peat soil-pozzolana substrate and grown at 18- 20°C, are treated at the cotyledon Z11 stage by spraying with the aqueous suspension described above. Plants, used as controls, are treated with an aqueous solution not containing the active material. After 24 hours, the plants are contaminated by depositing drops of an aqueous suspension of *Botrytis cinerea* spores (150,000 spores per ml) on upper surface of the leaves. The spores are collected from a 15-day-old culture and are suspended in a nutrient solution composed of:
- 20 g/L of gelatin
- 50 g/L of cane sugar
- 2 g/L of NH4NO3
- 1 g/L of KH2PO4

The contaminated cucumber plants are settled for 5/7 days in a climatic room at 15-11°C (day/night) and at 80% relative humidity.
Grading is carried out 5/7 days after the contamination, in comparison with the control plants. Under these conditions, good (at least 50%) protection is observed, at a dose of 500 g/ha, with a number of compounds of the present invention.

## Claims

1. Compounds of general formula (I): 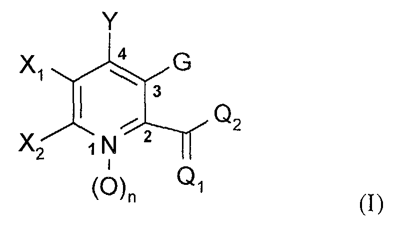 in which:
• Y and G together with carbon atoms 3 and 4, form a 5 or 6 member ring chosen from the following structures A to O : 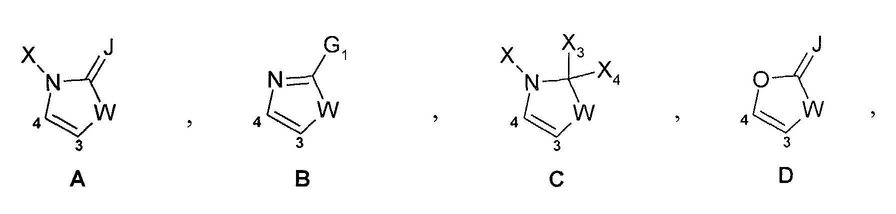 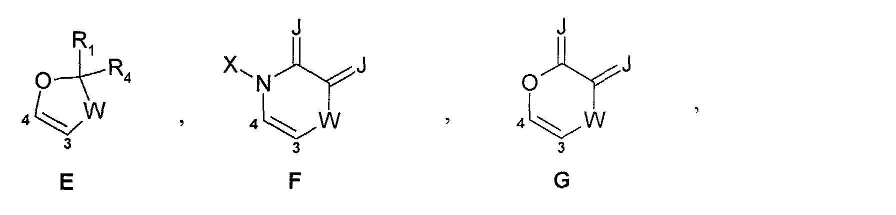 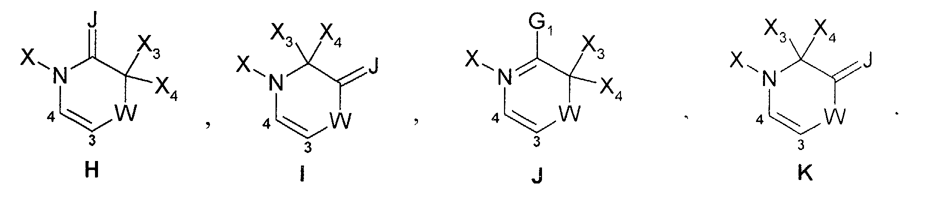 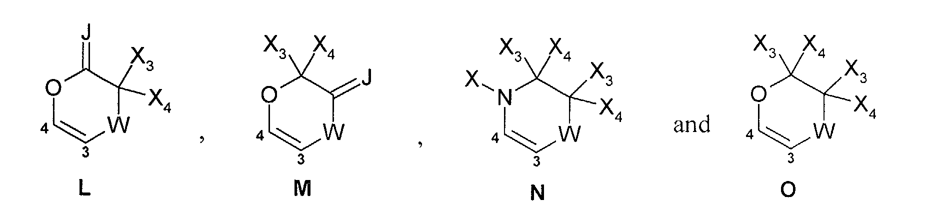
in which:
• J independently represents oxygen or sulphur,
• n represents 0 or 1,
• W is chosen from oxygen and sulphur,
• Q₁ is chosen from oxygen, sulphur, -NR₁ and -N-NR₄R₅,
• Q₂ is chosen from -OR₂, -SR₃ and -NR₄R₅, or
• Q₁ and Q₂ may together form a ring having from 5 to 7 atoms containing 2 to 3 oxygen and/or nitrogen atoms, optionally substituted with one or more radicals, which may be identical or different, chosen from halogens and alkyl and haloalkyl radicals,
• G₁ is chosen from hydrogen, halogen, hydroxy, mercapto, nitro, thiocyanato. cyano or pentafluorosulphonyl, alkyl, haloalkyl, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, 1, haloalkylsulphonyl, alkoxysulphonyl, cycloalkyl, halocycloalkyl, alkenyl, alkynyl, alkenyloxy, alkynyloxy, alkenylthio, alkynylthio, allyl, aryl, arylalkyl, propargyl, amino, N-alkylamino, N,N-dialkylamino, -NHCOR₁₀, -NHCSR₁₀, N-alkylaminocarbonylamino, N,N-dialkylaminocarbonylamino, aminoalkyl, N-alkylaminoalkyl, N,N-dialkylaminoalkyl, acylaminoalkyl, thioacylamino, alkoxythiocarbonylamino, N-alkylaminothiocarbonylamino, N,N-dialkylaminothiocarbonylamino, N,N-arylalkylaminocarbonylamino, N-alkylsulphinylamino, N-alkylsulphonylamino, N-alkyl(alkylsulphonyl)amino, N-arylsulphinylamino, N-arylsulphonylamino, N-alkoxysulphonylamino, N-alkoxysulphinylamino, N-haloalkoxysulphinylamino, N-haloalkoxysulphonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino, N,N-arylalkylaminothiocarbonylamino, acyl, carboxyl, carbamoyl, N-alkylcarbamoyl, N,N-dialkylcarbamoyl, lower alkoxycarbonyl, N-arylcarbamoyl, N,N-diarylcarbamoyl, aryloxycarbonyl, N,N-arylalkylcarbamoyl, and an imino group of formula: 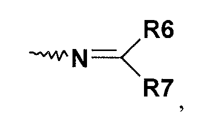
• X is chosen from hydrogen, alkyl, haloalkyl, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl, alkoxysulphonyl, cycloalkyl, halocycloalkyl, alkenyl, alkynyl, alkenyloxy, alkynyloxy, alkenylthio, alkynylthio, allyl, aryl, arylalkyl, propargyl, acyl, carboxyl, carbamoyl, aryloxycarbonyl,
• X₁ and X₂ are identical or different and chosen, independently of each other, from hydrogen, halogen, hydroxy, mercapto, nitro, thiocyanato, azido, cyano, pentafluorosulphonyl, alkyl, haloalkyl, alkoxy, haloalkoxy, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulphinyl, haloalkylsulphinyl, alkylsulphonyl, haloalkylsulphonyl, and alkoxysulphonyl, or
• X₁ and X₂ may also be joined together, thus forming a saturated, partially unsaturated or totally unsaturated 4- to 8-membered ring optionally comprising one or more heteroatoms chosen from sulphur, oxygen, nitrogen and phosphorus,
• X₃, X₄ are identical or different and chosen, independently of each other, from hydrogen, optionally substituted alkyl comprising from 1 to 12 carbon atoms in a linear or branched chain, hydroxy, amino, haloalkyl, cycloalkyl, halocycloalkyl, alkoxy, aryloxy, arylalkoxy, haloalkoxy, alkylthio, haloalkylthio, alkoxyalkyl, haloalkoxyalkyl, alkylthioalkyl, haloalkylthioalkyl, cyanoalkyl, acyl, nitro, cyano, carboxyl, carbamoyl, 3-oxetanyloxycarbonyl, N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl, N,N-dialkylaminoalkyl radical, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
• X₃ and X₄ may be joined together, thus forming a saturated, partially unsaturated or totally unsaturated 4- to 8-membered ring optionally comprising one or more heteroatoms chosen from sulphur, oxygen, nitrogen and phosphorus,
• R₂ and R₃ are identical or different and chosen, independently of each other, from alkyl comprising from 1 to 12 carbon atoms, haloalkyl, cycloalkyl, halocycloalkyl, alkoxy, haloalkoxy, alkylthio, haloalkylthio, alkoxyalkyl, haloalkoxyalkyl, alkylthioalkyl, haloalkylthioalkyl, cyanoalkyl, acyl, nitro, cyano, carboxyl, carbamoyl, 3-oxetanyloxycarbonyl, and N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl. alkynyl, N-alkylamino, N,N-dialkylamino, N-alkylaminoalkyl, and N,N-dialkylaminoalkyl radical, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
• R₁, R₄, R₅, R₆ and R₇ are identical or different and chosen, independently of each other, from hydrogen, optionally substituted alkyl comprising from 1 to 12 carbon atoms in a linear or branched chain, haloalkyl, cycloalkyl, halocycloalkyl, alkoxy, aryloxy, arylalkoxy, haloalkoxy, alkylthio, haloalkylthio, alkoxyalkyl, haloalkoxyalkyl, alkylthioalkyl, haloalkylthioalkyl, cyanoalkyl, acyl, nitro, cyano, carboxyl, carbamoyl, 3-oxetanyloxycarbonyl, and N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl. alkynyl, N-alkylamino, N,N-dialkylamino, N-alkylaminoalkyl, N,N-dialkylaminoalkyl, and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
• R₄ and R₅, on the one hand, or R₆ and R₇, on the other hand, may be joined together, thus forming a saturated, partially unsaturated or totally unsaturated 4-to 8-membered ring optionally comprising one or more heteroatoms chosen from sulphur, oxygen, nitrogen and phosphorus,
• T represents a direct bond or a divalent radical chosen from a radical -(CH₂)ₘ-, m taking a value between 1 and 12, limits included, said radical being optionally interrupted or ending with one or two heteroatoms chosen from nitrogen, oxygen and/or sulphur, and an oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O- vinyl (-C=C-), ethynyl (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- or -O-NR₉-CO- radical,
• R₈ is chosen from hydrogen and aryl and heterocyclyl,
• R₉ and R₁₀, which may be identical or different, are chosen, independently of each other, from hydrogen, halogen, hydroxy, mercapto, nitro, thiocyanato, azido, cyano, pentafluorosulphonyl, alkyl, haloalkyl, alkoxy, haloalkoxy, alkylthio, haloalkylthio, alkoxyalkyl, haloalkoxyalkyl, alkylthioalkyl, haloalkylthioalkyl, arylalkyl, cyanoalkyl, cyanoalkoxy, cyanoalkylthio, alkylsulphinyl, haloalkylsulphinyl, alkylsulphonyl, haloalkylsulphonyl, and alkoxysulphonyl,
as well as any optional N-oxides, geometrical and/or optical isomers, enantiomers and/or diastereoisomers, tautomeric forms, salts and metal and metalloid complexes of the compounds of formula (I) as here-above defined.

2. Compounds according to Claim 1 for which:
• X₁ and X₂ each represent a hydrogen atom,
• the other substituants being as defined in Claim 1,
as well as the optional N-oxides, geometrical and/or optical isomers, enantiomers and/or diastereoisomers, tautomeric forms, salts and metal and metalloid complexes thereof.

3. Compounds according to Claim 1 or 2, for which:
• Q₁ is chosen from oxygen and sulphur,
• the other substituants being as defined in Claim 1,
as well as optional N-oxides, geometrical and/or optical isomers, enantiomers and/or diastereoisomers, tautomeric forms, salts and metal and metalloid complexes thereof.

4. Compounds according to any of Claims 1 to 3, for which:
• Y and G together with carbon 3 and 4, form a ring chosen from structures A to E as described herein before,
• the other substituants being as defined above,
as well as the optional N-oxides, geometrical and/or optical isomers, enantiomers and/or diastereoisomers, tautomeric forms, salts and metal and metalloid complexes thereof.

5. Compounds according to any of the preceding Claims, for which:
• Y and G together with carbon 3 and 4, forms a 5 member ring chosen from : 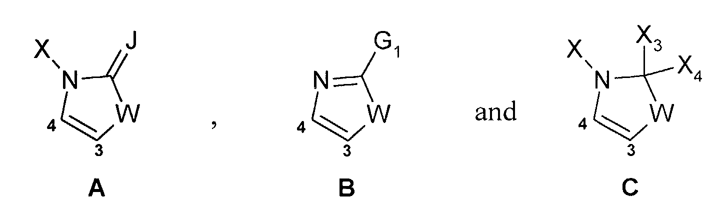
• the other substituants being as defined above,
as well as the optional N-oxides, geometrical and/or optical isomers, enantiomers and/or diastereoisomers, tautomeric forms, salts and metal and metalloid complexes thereof.

6. Compounds according to any of the preceding Claims, for which:
• Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl, alkynyl radical and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
• the other substituants being as defined above,
as well as the optional N-oxides, geometrical and/or optical isomers, enantiomers and/or diastereoisomers, tautomeric forms, salts and metal and metalloid complexes thereof.

7. Compounds according to Claim 1 having the following characteristics, taken individually or in combination:
• X₁ and X₂ each represent hydrogen,
• Y and G together with carbon 3 and 4, forms a 5 member ring chosen from : 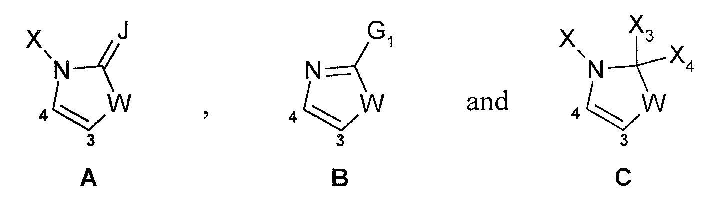
• Q₁ is chosen from oxygen and sulphur,
• Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl and alkynyl radical and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
• the other substituants being as defined above, as well as the optional N-oxides, geometrical and/or optical isomers, enantiomers and/or diastereoisomers, tautomeric forms, salts and metal and metalloid complexes thereof.

8. Compounds according to any of the preceding Claims, having the following characteristics:
• X₁ and X₂ each represent hydrogen,
• Y and G together with carbon 3 and 4, forms a 5 member ring chosen from : 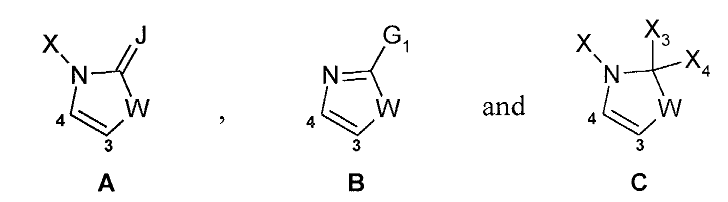
• G1 is chosen from alkyl, hydrogen, and -N,N-dialkylamino,
• Q₁ is chosen from oxygen and sulphur,
• Q₂ represents -NR₄R₅, in which R₄ represents hydrogen and R₅ is chosen from optionally substituted alkyl comprising from 1 to 12 carbon atoms in a linear or branched chain, haloalkyl, alkoxy, haloalkoxy, alkylthio, haloalkylthio, alkenyl and alkynyl and a radical chosen from aryl, arylalkyl, heterocyclyl and heterocyclylalkyl, optionally substituted with one or more radicals R₉ and/or aryl and/or arylalkyl, which may be identical or different, and/or a group -T-R₈,
• the other substituants being as defined above,
as well as the optional N-oxides, geometrical and/or optical isomers, enantiomers and/or diastereoisomers, tautomeric forms, salts and metal and metalloid complexes thereof.

9. Process for preparing the compounds according to one of Claims 1 to 8, in which Y and G, together with carbon 3 and 4 form ring A as defined in Claim 1, **characterised in that** a compound of formula (II): 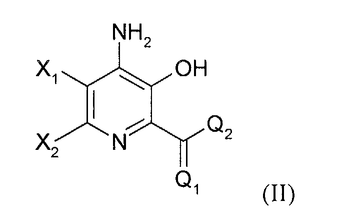 in which Q₁, Q₂, X₁ and X₂ are as defined in Claim 1,
is reacted with a phosgene or thiophosgene solution, this reaction being carried out in an apolar aprotic solvent, at reflux or at a temperature of between 20°C and 200°C.

10. Process for preparing the compounds according to one of Claims 1 to 8, in which Y and G, together with carbon 3 and 4 form ring B as defined in Claim 1, **characterised in that** a compound of formula (II): 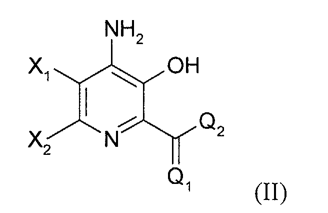 in which Q₁, Q₂, X₁ and X₂ are as defined in Claim 1,
is reacted with acid chloride, this reaction being carried out in an apolar aprotic solvent, at reflux or at a temperature of between 20°C and 200°C.

11. Process for preparing the compounds according to one of Claims I to 8, in which Y and G, together with carbon 3 and 4 form ring C as defined in Claim 1, **characterised in that** a compound of formula (II): 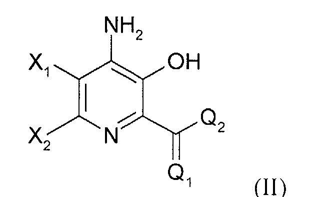 in which Q₁, Q₂, X₁ and X₂ are as defined in Claim 1,
is reacted with an alkyl- or an aryl ketone, this reaction being carried out in an apolar aprotic solvent, at reflux or at a temperature of between 20°C and 200°C.

12. Fungicidal compositions, comprising, as active material, an effective amount of at least one compound according to any one of Claims 1 to 8 or one of the N-oxides, geometrical and/or optical isomers, enantiomers and/or diastereoisomers, tautomeric forms, salts and metal and metalloid complexes thereof, that are agriculturally acceptable.

13. Fungicidal compositions according to Claim 12, comprising, besides the active material according to any one of Claims 1 to 8 or one of the N-oxides, geometrical and/or optical isomers, enantiomers and/or diastereoisomers, tautomeric forms, salts and metal and metalloid complexes thereof, that are agriculturally acceptable, an agriculturally acceptable solid or liquid support and/or a surfactant which is also agriculturally acceptable, as well as, optionally, one or more other fungicides, insecticides, herbicides, acaricides, attractants or pheromones and other compounds with biological activity.

14. Fungicidal compositions according to either of Claims 12 and 13, comprising from 0.05% to 99% by weight of active material.

15. Process for preventively or curatively combating the phytopathogenic fungi of crops, **characterised in that** an effective (agronomically effective) and non-phytotoxic amount of an active material of formula (I) according to one of Claims 1 to 8, or of a fungicidal composition comprising an active material of formula (I), is applied to the plant seeds or to the plant leaves and/or to the fruits of the plants or to the soil in which the plants are growing or in which it is desired to grow them.

16. Process according to Claim 15, in which rice, corn, cotton, cereals, fruit trees, oil-yielding crops, market-garden and vegetable crops, tomato, lettuce, protein-yielding crops, pea, solanacea plants, beetroot, flax and woodland trees, as well as genetically modified homologues of these crops, are treated.

17. Process according to either of Claims 15 and 16, in which the seeds of cereals, rice, corn, cotton, fruit trees, woodland trees, oil-yielding crops, protein-yielding crops, market-garden crops, solanacea plants, beetroot or flax, as well as genetically modified homologues of these crops, are treated.

18. Process according to any one of Claims 15 to 17, **characterised in that** the plants are genetically modified plants.

19. Process according to any one of Claims 15 to 18, in which the dose of active material applied is between 10 g and 800 g of active material per hectare, in the case of foliar treatments.

20. Process according to Claim 19, in which the dose of active material applied is between 50 g and 300 g of active material per hectare in the case of foliar treatments.

21. Process according to any one of Claims 15 to 18, in which the dose of active material applied is between 2 and 200 g of active material per 100 kg of seed, in the case of seed treatments.

22. Process according to Claim 21, in which the dose of active material applied is between 3 g and 150 g per 100 kg of seed, in the case of seed treatments.

23. Method for preventively or curatively treating lumber using the compounds according to one of Claims 1 to 8 or compositions containing them, against fungal attack, by direct application, sprinkling, dipping or injection.

24. Use of the compounds according to one of Claims 1 to 8 in human and animal therapy for the treatment of fungal diseases.
